# EUROPEAN PATENT APPLICATION

(11) **EP 4 249 000 A2**
(43) Date of publication of application: **27.09.2023**
(21) Application number: 23185461.3
(22) Date of filing: 10.02.2020
(51) Int. Cl.: A61K 45/06

(54) **PHARMACEUTICAL COMBINATION COMPRISING TNO155 AND A KRASG12C INHIBITOR**

(30) Priority: 12.02.2019 US 201962804706 P
(62) Divisional of application: 20707825.4
(71) Applicant: Novartis AG, 4056 Basel (CH)
(72) Inventor: BRACHMANN, Saskia Maria, 4002 Basel (CH); MADDALO, Danilo, 4002 Basel (CH); PRAHALLAD, Anirudh Cadapa, 4002 Basel (CH)
(74) Representative: Ridout, Joseph

(57) **Abstract**

The present invention relates to a pharmaceutical combination comprising TNO155 and a KRASG12C inhibitor; pharmaceutical compositions comprising the same; and methods of using such combinations and compositions in the treatment or prevention of conditions in a SHP2 inhibitor combined with KRASG12C inhibition is beneficial in, for example, the treatment of cancers.

## Description

### FIELD OF THE INVENTION

The present invention relates to a pharmaceutical combination comprising TNO155 and a KRASG12C inhibitor; pharmaceutical compositions comprising the same; and methods of using such combinations and compositions in the treatment or prevention of conditions in which SHP2 inhibition combined with KRASG12C inhibition is beneficial, for example, in the treatment of cancers.

### BACKGROUND OF THE INVENTION

TNO155 is an orally bioavailable, allosteric inhibitor of Src homology-2 domain containing protein tyrosine phsophatase-2 (SHP2, encoded by the *PTPN11* gene), which transduces signals from activated receptor tyrosine kinases (RTKs) to downstream pathways, including the mitogen-activated protein kinase (MAPK) pathway. SHP2 has also been implicated in immune checkpoint and cytokine receptor signaling. TNO155 has demonstrated efficacy in a wide range of RTK-dependent human cancer cell lines and *in vivo* tumor xenografts.

The Ras proteins are critical components of signalling pathways that direct cell growth, differentiation, proliferation and survival. RAS genes are frequently mutated oncogenes in human cancers, with approximately 30% of all human cancers have a mutation in KRAS, NRAS or HRAS genes. Oncogenic Ras is associated with mutations at glycine 12, glycine 13 or glutamine 61 of Ras. These residues are located at the active site of Ras and mutations result in aberrant activation of down-stream effector pathways (MAPK and PI3K pathways). KRAS is the most frequently mutated RAS gene in cancer with several tumor types exhibiting a high frequency of activating mutations in KRAS including: pancreatic (-90% prevalence); colorectal (-40% prevalence); and non-small cell lung cancer (-30% prevalence). KRAS mutations can be found in other cancer types including multiple myeloma, uterine cancer, bile duct cancer, stomach cancer, bladder cancer, diffuse large B cell lymphoma, rhabdomyosarcoma, cutaneous squamous cell carcinoma, cervical cancer and testicular germ cell cancer.

The G12C mutation is commonly found in RAS genes that accounts for 14% of all KRAS, 2% of all NRAS and 2% of all HRAS mutations across cancer types. The G12C mutation is particularly enriched in KRAS mutant non-small cell lung cancer with approximately half carrying this mutation. The G12C mutation is not exclusively associated with lung cancer and is found in other RAS mutant cancer types including 8% of all KRAS mutant colorectal cancer.

The combination of the present invention, TNO155 and a KRASG12C inhibitor, shows improved efficacy compared to either single agent alone in the treatment of, for example, esophageal or head and neck squamous cell carcinoma, colorectal, ovarian, pancreatic or non-small cell lung cancer, and renal cell carcinoma.

### SUMMARY OF THE INVENTION

The present invention provides for a pharmaceutical combination comprising:
(a) a SHP2 inhibitor selecte from (3S,4S)-8-(6-amino-5-((2-amino-3-chloropyridin-4-yl)thio)pyrazin-2-yl)-3-methyl-2-oxa-8-azaspiro[4.5]decan-4-amine (TNO155), or a pharmaceutically acceptable salt thereof, having the structure: 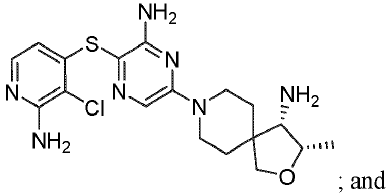 6-(4-amino-4-methylpiperidin-1-yl)-3-(2,3-dichlorophenyl)pyrazin-2-amine (SHP099), or a pharmaceutically acceptable salt thereof, having the structure: 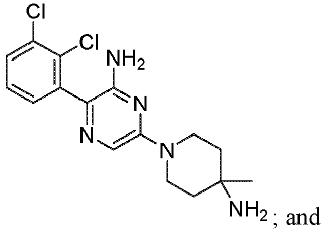
(b) a KRASG12C inhibitor.

Combinations of TNO155, or a pharmaceutically acceptable salt thereof, and a KRASG12C inhibitor, or a pharmaceutically acceptable salt thereof, will also be referred to herein as a "combination of the invention".

In another embodiment of the combination of the invention, TNO155 or a pharmaceutically acceptable salt thereof and a KRASG12C inhibitor, or a pharmaceutically acceptable salt thereof, are in the same formulation.

In another embodiment of the combination of the invention, TNO155 or a pharmaceutically acceptable salt thereof and a KRASG12C inhibitor, or a pharmaceutically acceptable salt thereof are in separate formulations.

In another embodiment, the combination of the invention is for simultaneous or sequential (in any order) administration.

In another embodiment is a method for treating or preventing cancer in a subject in need thereof comprising administering to the subject a therapeutically effective amount of the combination of the invention.

In a further embodiment of the method, the cancer is selected from: esophageal or head and neck squamous cell carcinoma; colorectal, ovarian, pancreatic or non-small cell lung cancer; and renal cell carcinoma.

In a further embodiment of the method, the cancer is selected from colorectal, ovarian, pancreatic and non-small cell lung cancer.

In a further embodiment of the method, the cancer is renal cell carcinoma.

In a further embodiment, the combination of the invention provides for a use in the manufacture of a medicament for treating a cancer selected from: esophageal or head and neck squamous cell carcinoma; colorectal, ovarian, pancreatic or non-small cell lung cancer; and renal cell carcinoma.

In another embodiment is a pharmaceutical composition comprising the combination of the invention.

In a further embodiment, the pharmaceutical composition further comprises one or more pharmaceutically acceptable excipients as detailed herein.

### BRIEF DESCRIPTION OF THE DRAWINGS

Figure 1: Combination of SHP2 and KRASG12C inhibitors (SHP099 and cmpd1, respectively) enhances growth inhibition in crystal violet cell growth assays in a panel of KRASG12C lung cancer cell lines.
Figure 2: Combination of SHP2 and KRASG12C inhibitors (SHP099 and cmpd1, respectively) enhances growth inhibition in crystal violet cell growth assays in a panel of KRASG12C lung cancer cell lines.
Figure 3: Combination of SHP2 and KRASG12C inhibitors (TNO155 and compound 2, respectively) enhances growth inhibition in crystal violet cell growth assays in a panel of KRASG12C lung cancer cell lines.
Figure 4: Combination of SHP2 and KRASG12C inhibitors (TNO155 and compound 3, respectively) enhances growth inhibition in crystal violet cell growth assays in a panel of KRASG12C lung cancer cell lines.
Figure 5: Combination of SHP2 and KRASG12C inhibitors (TNO155 and cmpd2, respectively) enhances tumor growth inhibition *in vivo* in the KRASG12C Miapaca-2 xenograft model.

### Definitions

The general terms used hereinbefore and hereinafter preferably have within the context of this disclosure the following meanings, unless otherwise indicated, where more general terms whereever used may, independently of each other, be replaced by more specific definitions or remain, thus defining more detailed embodiments of the invention:

A "KRASG12C inhibitor" is a compound selected from the compounds detailed in WO2013/155223, WO2014/143659, WO2014/152588, WO2014/160200, WO2015/054572, WO2016/044772, WO2016/049524, WO2016164675, WO2016168540, WO2017/058805, WO2017015562, WO2017058728, WO2017058768, WO2017058792, WO2017058805, WO2017058807, WO2017058902, WO2017058915, WO2017087528, WO2017100546, WO2017/201161, WO2018/064510, WO2018/068017, WO2018/119183, WO2018/217651, WO2018/140512, WO2018/140513, WO2018/140514, WO2018/140598, WO2018/140599, WO2018/140600, WO2018/143315, WO2018/206539, WO2018/218070, WO2018/218071, WO2019/051291, WO2019/099524, WO2019/110751, WO2019/141250, WO2019/150305, WO2019/155399, WO2019/213516, WO2019/213526, WO2019/217307 and WO2019/217691. Examples are: 1-(4-(6-chloro-8-fluoro-7-(3-hydroxy-5-vinylphenyl)quinazolin-4-yl)piperazin-1-yl)prop-2-en-1-one--methane (1/2) (compound 1); (S)-1-(4-(6-chloro-8-fluoro-7-(2-fluoro-6-hydroxyphenyl)quinazolin-4-yl)piperazin-1-yl)prop-2-en-1-one (compound 2); and 2-((S)-1-acryloyl-4-(2-(((S)-1-methylpyrrolidin-2-yl)methoxy)-7-(naphthalen-1-yl)-5,6,7,8-tetrahydropyrido[3,4-d]pyrimidin-4-yl)piperazin-2-yl)acetonitrile (compound 3).

The term "subject" or "patient" as used herein is intended to include animals, which are capable of suffering from or afflicted with a cancer or any disorder involving, directly or indirectly, a cancer. Examples of subjects include mammals, e.g., humans, apes, monkeys, dogs, cows, horses, pigs, sheep, goats, cats, mice, rabbits, rats, and transgenic non-human animals. In an embodiment, the subject is a human, e.g., a human suffering from, at risk of suffering from, or potentially capable of suffering from cancers.

The term "treating" or "treatment" as used herein comprises a treatment relieving, reducing or alleviating at least one symptom in a subject or effecting a delay of progression of a disease. For example, treatment can be the diminishment of one or several symptoms of a disorder or complete eradication of a disorder, such as cancer. Within the meaning of the present disclosure, the term "treat" also denotes to arrest, delay the onset (i.e., the period prior to clinical manifestation of a disease) and/or reduce the risk of developing or worsening a disease.

The terms "comprising" and "including" are used herein in their open-ended and non-limiting sense unless otherwise noted.

The terms "a" and "an" and "the" and similar references in the context of describing the invention (especially in the context of the following claims) are to be construed to cover both the singular and the plural, unless otherwise indicated herein or clearly contradicted by context. Where the plural form is used for compounds, salts, and the like, this is taken to mean also a single compound, salt, or the like.

The term "combination therapy" or "in combination with" refers to the administration of two or more therapeutic agents to treat a condition or disorder described in the present disclosure (e.g., cancer). Such administration encompasses co-administration of these therapeutic agents in a substantially simultaneous manner, such as in a single capsule having a fixed ratio of active ingredients. Alternatively, such administration encompasses co-administration in multiple, or in separate containers (e.g., capsules, powders, and liquids) for each active ingredient. Powders and/or liquids may be reconstituted or diluted to a desired dose prior to administration. In addition, such administration also encompasses use of each type of therapeutic agent in a sequential manner, either at approximately the same time or at different times. In either case, the treatment regimen will provide beneficial effects of the drug combination in treating the conditions or disorders described herein.

The combination therapy can provide "synergy" and prove "synergistic", *i.e*., the effect achieved when the active ingredients used together is greater than the sum of the effects that results from using the compounds separately. A synergistic effect can be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect can be attained when the compounds are administered or delivered sequentially, *e.g*., by different injections in separate syringes. In general, during alternation therapy, an effective dosage of each active ingredient is administered sequentially, *i.e*., serially, whereas in combination therapy, effective dosages of two or more active ingredients are administered together.

The term "pharmaceutical combination" as used herein refers to either a fixed combination in one dosage unit form, or non-fixed combination or a kit of parts for the combined administration where two or more therapeutic agents may be administered independently at the same time or separately within time intervals, especially where these time intervals allow that the combination partners show a cooperative, *e.g*. synergistic effect.

The term "synergistic effect" as used herein refers to action of two therapeutic agents such as, for example, a compound TNO155 as a SHP2 inhibitor and a KRASG12C inhibitor, producing an effect, for example, slowing the symptomatic progression of a proliferative disease, particularly cancer, or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

The combination of the invention, TNO155 andKRASG12C inhibitor, is also intended to represent unlabeled forms as well as isotopically labeled forms of the compounds. Isotopically labeled compounds have one or more atoms replaced by an atom having a selected atomic mass or mass number. Examples of isotopes that can be incorporated into TNO155 and a KRASG12C inhibitor include isotopes of hydrogen, carbon, nitrogen, oxygen, and chlorine, for example, ²H, ³H, ¹¹C, ¹³C, ¹⁴C, ¹⁵N, ³⁵S, ³⁶Cl. The invention includes isotopically labeled TNO155 and a KRASG12C inhibitor, for example into which radioactive isotopes, such as ³H and ¹⁴C, or non-radioactive isotopes, such as ²H and ¹³C, are present. Isotopically labelled TNO155 and a KRASG12C inhibitor are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. Isotopically-labeled compounds of the invention can generally be prepared by conventional techniques known to those skilled in the art or by processes analogous to those described in the accompanying Examples using appropriate isotopically-labeled reagents.

Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of either TNO155 or a KRASG12C inhibitor. The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in TNO155 or a KRASG12C inhibitor is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

### Description of Preferred Embodiments

TNO155 is an investigational agent that is an orally bioavailable small molecule inhibitor of SHP2 activity. SHP2 transduces signaling downstream of activated RTKs. In preclinical models, tumor dependence on RTKs predicts dependence on SHP2.

In one embodiment is a method of treating cancer comprising adminstering to a subject in need thereof a pharmaceutical composition comprising (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, in combination with a second therapeutic agent.

In a further embodiment, the cancer is selected from: esophageal or head and neck squamous cell carcinoma; colorectal, ovarian, pancreatic or non-small cell lung cancer; and renal cell carcinoma.

In a further embodiment of the method, the cancer is colorectal cancer.

In a further embodiment of the method, the cancer is non-small cell lung cancer.

In a further embodiment of the method, the cancer is head and neck squamous cell carcinoma.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent are are administered simultaneously, separately or over a period of time.

In a further embodiment, the amount of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, administered to the subject in need therof is effective to treat the cancer.

In a further embodiment, the amount of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent, administered to the subject in need therof, is effective to treat the cancer.

In a further embodiment, the second therapeutic agent is a KRASG12C inhibitor.

In a further embodiment, the KRASG12C inhibitor is selected from 1-(4-(6-chloro-8-fluoro-7-(3-hydroxy-5-vinylphenyl)quinazolin-4-yl)piperazin-1-yl)prop-2-en-1-one-methane (1/2) (compound 1), (S)-1-(4-(6-chloro-8-fluoro-7-(2-fluoro-6-hydroxyphenyl)quinazolin-4-yl)piperazin-1-yl)prop-2-en-1-one (compound 2), and 2-((S)-1-acryloyl-4-(2-(((S)-1-methylpyrrolidin-2-yl)methoxy)-7-(naphthalen-1-yl)-5,6,7,8-tetrahydropyrido[3,4-d]pyrimidin-4-yl)piperazin-2-yl)acetonitrile (compound 3), or a pharmaceutically acceptable salt thereof.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day, or 70 mg per day, or 80 mg per day, or 90 mg per day, or 100 mg per day.

In a further embodiment, the dose per day of (3S,4S)-8-(6-amino-5-((2-amino-3-chloropyridin-4-yl)thio)pyrazin-2-yl)-3-methyl-2-oxa-8-azaspiro[4.5]decan-4-amine is on a 21 day cycle of 2 weeks on drug followed by 1 week off drug.

In a further embodiment, the dose per day of (3S,4S)-8-(6-amino-5-((2-amino-3-chloropyridin-4-yl)thio)pyrazin-2-yl)-3-methyl-2-oxa-8-azaspiro[4.5]decan-4-amine is 20 mg.

In a further embodiment the dosing schedule is once daily (QD) or twice daily (BID).

In another embodiment is a method of treating cancer comprising administering, to a patient in need thereof, (3S,4S)-8-(6-amino-5-((2-amino-3-chloropyridin-4-yl)thio)pyrazin-2-yl)-3-methyl-2-oxa-8-azaspiro[4.5]decan-4-amine is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day, or 70 mg per day, or 80 mg per day, or 90 mg per day, or 100 mg per day.

In a further embodiment, the dose per day of (3S,4S)-8-(6-amino-5-((2-amino-3-chloropyridin-4-yl)thio)pyrazin-2-yl)-3-methyl-2-oxa-8-azaspiro[4.5]decan-4-amine is on a 21 day cycle of 2 weeks on drug followed by 1 week off drug.

In a further embodiment, the dose per day of (3S,4S)-8-(6-amino-5-((2-amino-3-chloropyridin-4-yl)thio)pyrazin-2-yl)-3-methyl-2-oxa-8-azaspiro[4.5]decan-4-amine is 20 mg.

In a further embodiment the dosing schedule is once daily (QD) or twice daily (BID).

In a further embodiment, the cancer is selected from: esophageal or head and neck squamous cell carcinoma; colorectal, ovarian, pancreatic or non-small cell lung cancer; and renal cell carcinoma.

In a further embodiment of the method, the cancer is colorectal cancer.

In a further embodiment of the method, the cancer is non-small cell lung cancer.

In a further embodiment of the method, the cancer is head and neck squamous cell carcinoma.

In a further embodiment, the method further comprises a second therapeutic agent.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent are are administered simultaneously, separately or over a period of time.

In a further embodiment, the second therapeutic agent is a KRASG12C inhibitor.

In a further embodiment, the KRASG12C inhibitor is selected from 1-(4-(6-chloro-8-fluoro-7-(3-hydroxy-5-vinylphenyl)quinazolin-4-yl)piperazin-1-yl)prop-2-en-1-one-methane (1/2) (compound 1), (S)-1-(4-(6-chloro-8-fluoro-7-(2-fluoro-6-hydroxyphenyl)quinazolin-4-yl)piperazin-1-yl)prop-2-en-1-one (compound 2), and 2-((S)-1-acryloyl-4-(2-(((S)-1-methylpyrrolidin-2-yl)methoxy)-7-(naphthalen-1-yl)-5,6,7,8-tetrahydropyrido[3,4-d]pyrimidin-4-yl)piperazin-2-yl)acetonitrile (compound 3), or a pharmaceutically acceptable salt thereof.

In one embodiment, with respect to the pharmaceutical combination of the invention, is a pharmaceutical combination comprising (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or a pharmaceutically acceptable salt thereof, and a KRASG12C inhibitor, or a pharmaceutically acceptable salt thereof, are administered separately, simultaneously or sequentially, in any order.

In a further embodiment, the pharmaceutical combination is for oral administration.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine is in an oral dose form.

In a further embodiment, the KRASG12C inhibitor is in an oral dose form.

In another embodiment, is a pharmaceutical composition comprising a pharmaceutical combination of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof and at least one pharmaceutically acceptable carrier.

In a further embodiment, is a pharmaceutical combination of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof, for use in the treatment of esophageal or head and neck squamous cell carcinoma.

In another embodiment, is a pharmaceutical combination of (3 S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof, for use in the treatment of colorectal, ovarian, pancreatic or non-small cell lung cancer.

In another embodiment, is a pharmaceutical combination of (3 S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof, for use in the treatment of renal cell carcinoma.

In another embodiment, is a use of the pharmaceutical combination of ((3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof, for the manufacture of a medicament for the treatment of a cancer selected from: esophageal or head and neck squamous cell carcinoma; colorectal, ovarian, pancreatic or non-small cell lung cancer; and renal cell carcinoma.

In another embodiment, is a method of treating a cancer selected from: esophageal or head and neck squamous cell carcinoma; colorectal, ovarian, pancreatic or non-small cell lung cancer; and renal cell carcinoma; comprising administrating to a patient in need thereof a pharmaceutical combination of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof, or a pharmaceutical composition comprising a pharmaceutical combination of (3 S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof and at least one pharmaceutically acceptable carrier.

In another embodiment, is a method of treating a cancer selected from: esophageal or head and neck squamous cell carcinoma; colorectal, ovarian, pancreatic or non-small cell lung cancer; and renal cell carcinoma; comprising administrating to a patient in need thereof a pharmaceutical combination of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof, or a pharmaceutical composition comprising a pharmaceutical combination of (3 S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a KRASG12C inhibitor, or a pharmaceutically acceptable salt thereof and at least one pharmaceutically acceptable carrier.

In another embodiment, (3S,4S)-8-(6-amino-5-((2-amino-3-chloropyridin-4-yl)thio)pyrazin-2-yl)-3-methyl-2-oxa-8-azaspiro[4.5]decan-4-amine is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day.

According to the invention, there is therefore provided the following numbered embodiments:
Embodiment 1. A method of treating cancer comprising adminstering to a subject in need thereof a pharmaceutical composition comprising (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, in combination with a second therapeutic agent.
Embodiment 2. The method of Embodiment 1, wherein the cancer is selected from: esophageal or head and neck squamous cell carcinoma; colorectal, ovarian, pancreatic or non-small cell lung cancer; and renal cell carcinoma.
Embodiment 3. The method according to Embodiment 1 or Embodiment 2, wherein (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent are are administered simultaneously, separately or over a period of time.
Embodiment 4. The method according to any one of Embodiments 1 to 3, wherein the amount of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, administered to the subject in need therof is effective to treat the cancer.
Embodiment 5. The method according to any one of Embodiments 1 to 4, wherein the amount of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent, administered to the subject in need therof, is effective to treat the cancer.
Embodiment 6. The method according to any one of Embodiments 1 to 5, wherein the second therapeutic agent is a KRASG12C inhibitor.
Embodiment 7. The method of Embodiment 6 wherein the KRASG12C inhibitor is selected from 1-(4-(6-chloro-8-fluoro-7-(3-hydroxy-5-vinylphenyl)quinazolin-4-yl)piperazin-1-yl)prop-2-en-1-one--methane (1/2) (compound 1), (S)-1-(4-(6-chloro-8-fluoro-7-(2-fluoro-6-hydroxyphenyl)quinazolin-4-yl)piperazin-1-yl)prop-2-en-1-one (compound 2), and 2-((S)-1-acryloyl-4-(2-(((S)-1-methylpyrrolidin-2-yl)methoxy)-7-(naphthalen-1-yl)-5,6,7,8-tetrahydropyrido[3,4-d]pyrimidin-4-yl)piperazin-2-yl)acetonitrile (compound 3), or a pharmaceutically acceptable salt thereof.
Embodiment 8. The method according to any one of Embodiments 1 to 7 wherein (3S,4S)-8-(6-amino-5-((2-amino-3-chloropyridin-4-yl)thio)pyrazin-2-yl)-3-methyl-2-oxa-8-azaspiro[4.5]decan-4-amine is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day.
Embodiment 9. The method of Embodiment 8 wherein the dose per day is on a 21 day cycle of 2 weeks on drug followed by 1 week off drug.
Embodiment 10. The method of Embodiment 9 wherein the dose per day is 20 mg QD.
Embodiment 11. A method of treating cancer comprising administering, to a patient in need thereof, (3S,4S)-8-(6-amino-5-((2-amino-3-chloropyridin-4-yl)thio)pyrazin-2-yl)-3-methyl-2-oxa-8-azaspiro[4.5]decan-4-amine is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day.
Embodiment 12. The method of Embodiment 11 wherein the dose per day is on a 21 day cycle of 2 weeks on drug followed by 1 week off drug.
Embodiment 13. The method of Embodiment 12 wherein the dose per day is 20 mg QD.
Embodiment 14. The method of Embodiment 11, wherein the cancer is selected from: esophageal or head and neck squamous cell carcinoma; colorectal, ovarian, pancreatic or non-small cell lung cancer; and renal cell carcinoma.
Embodiment 15. The method of Embodiment 11 further comprising a second therapeutic agent.

### Pharmacology and Utility

Non-small cell lung cancer - In 2012, approximately 1.8 million people worldwide were diagnosed with lung cancer, and an estimated 1.6 million people died from the disease. Non-small cell lung cancer comprises approximately 85% of lung cancers, with adenocarcinomas and squamous cell carcinomas being the most common subtypes. Standard of care treatment for advanced stage non-small cell lung carcinomas (NSCLCs) that do not harbor genetic alterations in druggable driver oncogenes such as EGFR, ALK, or ROS includes chemotherapy and immunotherapy, administered concurrently or sequentially. While these treatments provide clinical benefit, the majority of patients experience disease progression within a year, and the prognosis for patients with advanced NSCLC remains poor. Immunotherapy for NSCLC with immune checkpoint inhibitors has demonstrated promise, with some NSCLC patients experiencing durable disease control for years. However, such long-term non-progressors are uncommon, and combination treatment strategies that can increase the proportion of patients responding to and achieving lasting remission with immunotherapy using checkpoint inhibitors are urgently needed. Activating mutations in the KRAS oncogene occur in approximately 30% of lung adenocarcinomas, and have been associated with poor outcome in some studies. No approved drugs target mutant KRAS directly, so standard of care for advanced stage KRAS-mutant NSCLC is also chemotherapy and immunotherapy as described above.

Head and neck squamous cell cancer - Squamous cell cancers are the most common cancers occurring in the head and neck, with an estimated worldwide incidence of approximately 686,000 for oropharyngeal and laryngeal cancers combined. Alcohol and tobacco use are the most common risk factors for head and neck squamous cell cancers (HNSCCs), with human papilloma virus (HPV) infection likely also playing a causative role. More than 90% of HNSCCs have overexpression of EGFR or its ligands. For patients with metastatic disease, standard systemic treatment includes platinum-based chemotherapy with or without cetuximab. Historically, median survival with systemic chemotherapy is approximately six months, with only approximately 20% of patients surviving one year. More recently, a survival benefit has been shown for nivolumab, an anti-programmed death-1 (PD-1) antibody, versus standard second-line single agent therapy (docetaxel, methotrexate, or cetuximab) in patients who had progressed on platinum-based chemotherapy. Still, the survival rate at one year for patients treated with nivolumab was only 36%. Therefore, a great need exists for improved treatments for this aggressive and debilitating cancer.

Colorectal cancer - Colorectal cancer (CRC) is the second most common cancer in women and the third most common cancer in men, accounting for an estimated 1.4 million new cancer cases worldwide in 2012. Chromosomal instability and microsatellite instability both play roles in the pathogenesis of CRC. Chromosomal instability is found in approximately 85% of sporadic colorectal cancers and is characterized by mutations in the Wnt pathway genes, APC and CTNNB1. KRAS mutations, occurring most commonly in codon 12 or 13, are present in approximately 45% of these cases and render anti-EGFR therapies ineffective. Microsatellite instability (MSI), arising due to defective DNA mismatch repair, is involved in approximately 15% of sporadic CRCs, as well as CRCs arising in Lynch syndrome due to a germline mutation of a mismatch repair gene. MSI-high CRCs tend to have a better prognosis than non-MSI-high CRC, and also have responded differently to some systemic therapies. Systemic therapy for metastatic CRC includes various agents used alone or in combination, including chemotherapies such as 5-Fluorouracil/leucovorin, capecitabine, oxaliplatin, and irinotecan; anti-angiogenic agents such as bevacizumab and ramucirumab; anti-EGFR agents including cetuximab and panitumumab for KRAS/NRAS wild-type cancers; and immunotherapies including nivolumab and pembrolizumab. Despite multiple active therapies, however, metastatic CRC remains incurable. While CRCs that are deficient in mismatch repair (MSI-high) exhibit high response rates to immune checkpoint inhibitor therapy, mismatch repair proficient CRCs do not. Since KRAS-mutant CRCs are typically mismatch repair proficient and are not candidates for anti-EGFR therapy, this subtype of CRC is particularly in need of improved therapies.

TNO155 is a first-in-class allosteric inhibitor of wild-type SHP2. SHP2 is a ubiquitously expressed non-receptor protein tyrosine phosphatase (PTP) composed of two N-terminal SH2 domains, a classic PTP domain, and a C-terminal tail. The phosphatase activity is auto-inhibited by the two SHP2 domains that bind to the PTP domain (closed conformation). Upon activation of receptor tyrosine kinases (RTKs), SHP2 is recruited to the plasma membrane where it associates with activated RTKs and a number of adaptor proteins to relay signaling by activating the RAS/MAPK pathway. TNO155 binds the inactive, or "closed" conformation of SHP2, thereby preventing its opening into the active conformation. This prevents the transduction of signaling from activated RTKs to the downstream RAS/MAPK pathway.

TNO155 has demonstrated efficacy in a wide range of RTK-dependent human cancer cell lines and in vivo xenografts. Preclinical *in vitro* and *in vivo* evaluation of TNO155 demonstrate selective and potent inhibition of the SHP2 phosphatase, in RTK-dependent human cancer models, for example, esophageal, HNSCC and NSCLC. SHP2 inhibition can be measured by assessing biomarkers within the MAPK signaling pathway, such as decreased levels of phosphorylated ERK1/2 (pERK) and downregulation of dual specificity phosphatase 6 (DUSP6) mRNA transcript. In the KYSE-520 (esophageal squamous cell carcinoma) and DETROIT-562 (pharyngeal squamous cell carcinoma) cancer cell lines, the *in vitro* pERK IC50's were 8 nM (3.4 ng/mL) and 35 nM (14.8 ng/mL) and the antiproliferation IC50's were 100 nM (42.2 ng/mL) and 470 nM (198.3 ng/mL), respectively. The antiproliferative effect of TNO155 was revealed to be most effective in cancer cell lines that are dependent on RTK signaling. *In vivo,* SHP2 inhibition by orally-administered TNO155 (20 mg/kg) achieved approximately 95% decrease in DUSP6 mRNA transcript in an EGFR-dependent DETROIT-562 cancer cell line and 47% regression when dosed on a twice-daily schedule. Dose fractionation studies, coupled with modulation of the tumor DUSP6 biomarker show that maximal efficacy is achieved when 50% PD inhibition is attained for at least 80% of the dosing interval.

The KRAS, NRAS and HRAS genes encode a set of closely related small GTPase proteins KRas, NRas and HRas, collectively referred to herein as Ras, that share 82-90% overall sequence identity. The Ras proteins are critical components of signalling pathways transmitting signals from cell-surface receptors to regulate cellular proliferation, survival and differentiation. Ras functions as a molecular switch cycling between an inactive GDP-bound state and an active GTP-bound state.

On binding to GTP, Ras undergoes a conformational change which enables its interaction and activation of effector proteins to regulate down-stream signalling pathways. The best characterised effector of Ras is the serine/threonine kinase Raf which regulates the activity of the mitogen-activate protein kinase (MAPK) pathway. The PI3K pathway is another important effector pathway down-stream of Ras with the class I phosphoinositide 3-kinases interacting with Ras.

RAS mutations are frequently found in cancer and approximately 30% of all human cancers have a mutation in KRAS, NRAS or HRAS genes. Oncogenic Ras is typically, but not exclusively, associated with mutations at glycine 12, glycine 13 or glutamine 61 of Ras. These residues are located at the active site of Ras and mutations impair GAP-mediated and intrinsic hydrolysis activity favoring the formation of GTP bound Ras and aberrant activation of down-stream effector pathways. KRAS is the most frequently mutated RAS gene in cancer followed by NRAS and then HRAS. There are several tumor types that exhibit a high frequency of activating mutations in KRAS including pancreatic (-90% prevalence), colorectal (-40% prevalence) and non-small cell lung cancer (-30% prevalence). KRAS mutations are also found in other cancer types including multiple myeloma, uterine cancer, bile duct cancer, stomach cancer, bladder cancer, diffuse large B cell lymphoma, rhabdomyosarcoma, cutaneous squamous cell carcinoma, cervical cancer, testicular germ cell cancer and others.

Glycine to cysteine mutations at residue 12 of Ras (the G12C mutation) are commonly found in RAS genes that accounts for 14% of all KRAS, 2% of all NRAS and 2% of all HRAS mutations across cancer types. The G12C mutation is particularly enriched in KRAS mutant non-small cell lung cancer with approximately half carrying this mutation, which has been associated with the DNA adducts formed by tobacco smoke. The G12C mutation is not exclusively associated with lung cancer and is found in other RAS mutant cancer types including 8% of all KRAS mutant colorectal cancer.

The epidermal growth factor receptor (EGFR) is an established critical therapeutic target in NSCLCs harboring activating EGFR mutations. Numerous trials with first (e.g. erlotinib, gefitinib) and second (e.g. afatinib, dacomitinib) generation EGFR inhibitors have been conducted in the EGFR-mutant advanced/unresectable NSCLC population, and have consistently demonstrated superior efficacy of EGFR tyrosine kinase inhibitors (TKIs) over chemotherapy in this population. Resistance to 1^{st} generation EGFR TKIs has been shown to arise through the development of an EGFR "gatekeeper" T790M mutation that impairs binding of the TKI, as well as by activation of alternative RTK pathways, including *MET* and *ERBB2* amplification. Clinical trials using 3^{rd} generation, irreversible EGFR inhibitors (e.g., osimertinib, rociletinib), which inhibit EGFR activating and gatekeeper mutations have demonstrated efficacy in EGFR T790M-mutant NSCLCs, highlighting their continued dependence on EGFR signaling. Emerging data from cancers that have become resistant to 3^{rd} generation inhibitors suggest that these cancers continue to select for activated RTK signaling, with resistance mutations in EGFR (C797S) as well as RTK amplifications (*MET, ERBB2, FGFR1*) having been described. Limited treatment options are available for patients whose cancers have developed resistance to 1^{st}/2^{nd} and 3^{rd} generation EGFR TKIs. Since SHP2 transduces EGFR signaling, and preclinical models have demonstrated a strong correlation between RTK dependence and SHP2 dependence, TNO155 is predicted to provide clinical benefit in these cancers whether resistance is driven by signaling from EGFR or another RTK.

More than 90% of head and neck cancers are characterized by overexpression or amplification of *EGFR*; amplification/overexpression of other RTKs, particularly FGFRs, and their ligands is also common. Inhibition of EGFR with cetuximab in advanced HNSCCs has also demonstrated clinical benefit, though disease control is not durable. The modest efficacy of EGFR inhibition in HNSCC may be related to compensatory signaling through other RTKs, which would be predicted to be abrogated by SHP2 inhibition with TNO155 treatment. In addition, preclinical testing identified head and neck cancer cells as the lineage with the highest frequency of sensitivity to SHP2 inhibition.

Patients with metastatic or unresectable RTK-driven cancers such as anaplastic lymphoma kinase (ALK)-rearranged NSCLC or stem cell factor receptor (KIT)-mutant gastrointestinal stromal tumor (GIST) derive benefit from molecules directly targeting these RTKs, but resistance to these agents invariably occurs. Mechanisms of resistance frequently include drug-resistant mutations in the targeted RTK and/or activation of bypass RTK pathways; in most cases, further treatment options are limited. Targeting SHP2 with TNO155 is a rational approach in such RTK-dependent cancers.

The preclinical data presented in the examples, below, provide in vitro and in vivo evidence that the combination of the SHP2 inhibitor, TNO155 and a KRASG12C inhibitor exert a significant combination benefit in multiple cancers.

### Pharmaceutical Compositions

In another aspect, the present invention provides pharmaceutically acceptable compositions which comprise a therapeutically-effective amount TNO155 and a KRASG12C inhibitor, formulated together with one or more pharmaceutically acceptable carriers (additives) and/or diluents. As described in detail below, the pharmaceutical compositions of the present invention may be specially formulated for administration in solid or liquid form, including those adapted for oral administration, for example, drenches (aqueous or non-aqueous solutions or suspensions), tablets, e.g., those targeted for buccal, sublingual, and systemic absorption, boluses, powders, granules, pastes for application to the tongue.

The phrase "therapeutically-effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (*e.g*., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

As set out above, certain embodiments of the present compounds may contain a basic functional group, such as amino or alkylamino, and are, thus, capable of forming pharmaceutically-acceptable salts with pharmaceutically-acceptable acids. The term "pharmaceutically-acceptable salts" in this respect, refers to the relatively non-toxic, inorganic and organic acid addition salts of compounds of the present invention. These salts can be prepared *in situ* in the administration vehicle or the dosage form manufacturing process, or by separately reacting a purified compound of the invention in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", J. Pharm. Sci. 66:1-19).

The pharmaceutically acceptable salts of the subject compounds include the conventional nontoxic salts or quaternary ammonium salts of the compounds, *e.g*., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like. The pharmaceutically acceptable salt of TNO155, for example, is succinate.

In other cases, the compounds of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared *in situ* in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge et al., *supra*)

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 0.1 per cent to about ninety-nine percent of active ingredient, preferably from about 5 per cent to about 70 per cent, most preferably from about 10 percent to about 30 percent.

In certain embodiments, a formulation of the present invention comprises an excipient selected from the group consisting of cyclodextrins, celluloses, liposomes, micelle forming agents, *e.g.,* bile acids, and polymeric carriers, *e.g.,* polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution, suspension or solid dispersion in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, trouches and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

The compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of the combination of the invention will be that amount of each compound which is the lowest dose effective to produce a therapeutic effect. Such an effective dose will generally depend upon the factors described above.

In another aspect, the present invention provides pharmaceutically acceptable compositions which comprise a therapeutically-effective amount of one or more of the subject compounds, as described above, formulated together with one or more pharmaceutically acceptable carriers (additives) and/or diluents.

### Examples

(3S,4S)-8-(6-amino-5-((2-amino-3-chloropyridin-4-yl)thio)pyrazin-2-yl)-3-methyl-2-oxa-8-azaspiro[4.5]decan-4-amine (TNO155) is synthesized according to example 69 of WO2015/107495. 6-(4-amino-4-methylpiperidin-1-yl)-3-(2,3-dichlorophenyl)pyrazin-2-amine (SHP099) is synthesized according to example 7 of WO2015/107493. 1-(4-(6-chloro-8-fluoro-7-(3-hydroxy-5-vinylphenyl)quinazolin-4-yl)piperazin-1-yl)prop-2-en-1-one--methane (1/2) (compound 1) and (S)-1-(4-(6-chloro-8-fluoro-7-(2-fluoro-6-hydroxyphenyl)quinazolin-4-yl)piperazin-1-yl)prop-2-en-1-one (compound 2) are synthesized according to the examples in WO2016/164675. 2-((S)-1-acryloyl-4-(2-(((S)-1-methylpyrrolidin-2-yl)methoxy)-7-(naphthalen-1-yl)-5,6,7,8-tetrahydropyrido[3,4-d]pyrimidin-4-yl)piperazin-2-yl)acetonitrile (compound 3) is synthesized according to the examples in WO2017/201161.

The utility of SHP2 inhibitors and KRASG12C inhibitors, as described herein can be evidenced by testing in the following examples.

### Example 1

Cells were grown in RPMI Glutamax + 10% FCS + 1% each: Sodium pyruvate, Hepes buffer. At day 1 cells were seeded into 6 well plates with indicated cell number. At day 2 compound treatment was started with indicated compound concentrations. Cells were re-fed every 3-4 days with fresh compound / media. Crystal violet staining was performed at indicated day. 200µL of formaldehyde (stock concentration 37.8%) was added to each well (on top of the 2ml cell media) and incubated for 10min at RT. Wells were emptied, rinsed at least once with 5mL of water and emptied again. 1mL of purple violet 0.1% water was added into each well and incubated for 15min at RT. Wells were emptied and rinsed at least twice with 2mL of water. Plates were tried, pictures were scanned with CanoScan4400F and saved as PDF.

### Example 2

Athymic nude mice were subcutaneously injected into the right flank with a 25G needle with 3 million MiaPaCa-2 cells suspended in 50% matrigel/HBSS. Tumor growth was followed by caliper measurement and expressed as cubic millimeters. Tumors were allowed to grow to a size between 200 and 300 cubic mm, thereafter animals were randomized into individual groups as following: Vehicle control (4 animals; MC:Tween80:Water (0.5:0.1:99.4)); TNO155 - 10 mg/kg qd po (4 animals); compound 2 - 50 mg/kg qd po (4 animals); compound 2 - 200 mg/kg qd po (4 animals); compound 2 - 50 mg/kg in combination with TNO155 10mg/kg (5 animals); pretreatment with TNO155 10 mg/kg followed by treatment with compound 2 - 50 mg/kg (6 animals). Combo: compounds are given at the same time. ComboP: TNO155 is given 3 hours before compound 2. Two weeks after treatment animals were euthanized and tumour samples harvested for further analysis 6h after the last treatment.

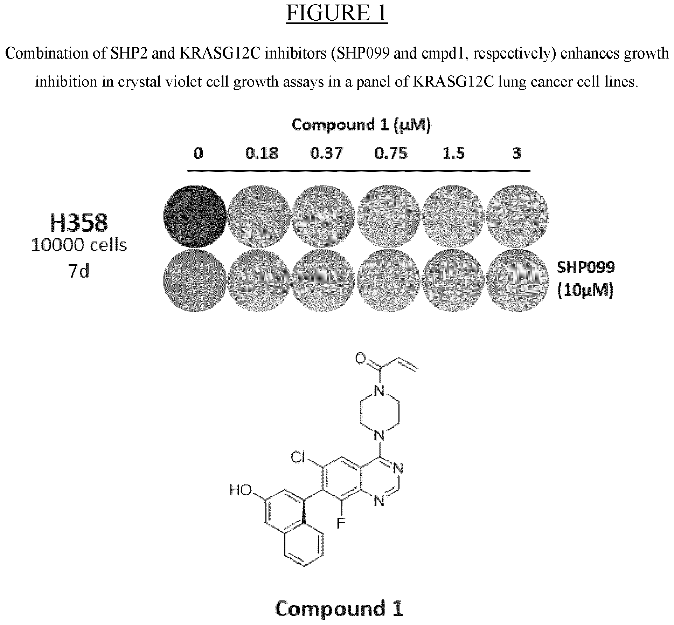

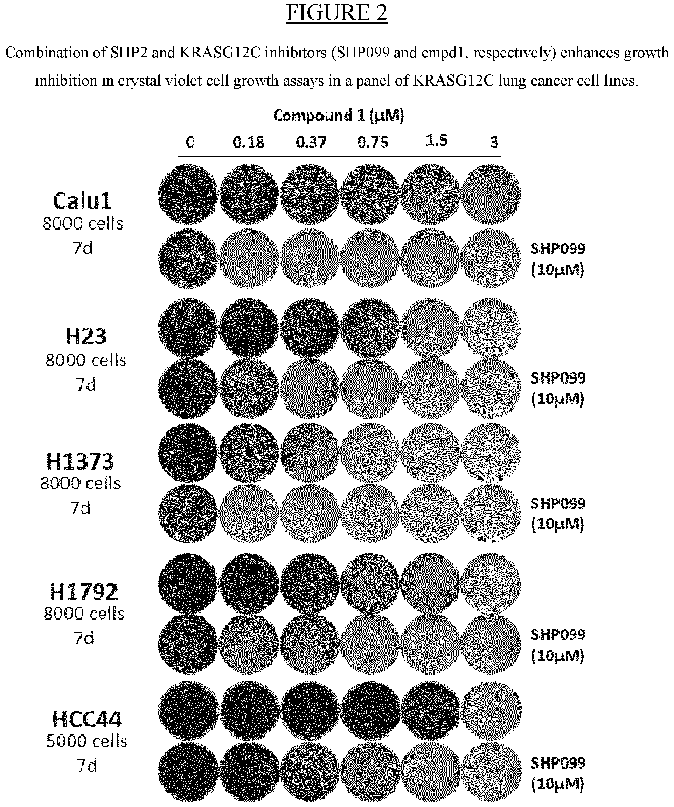

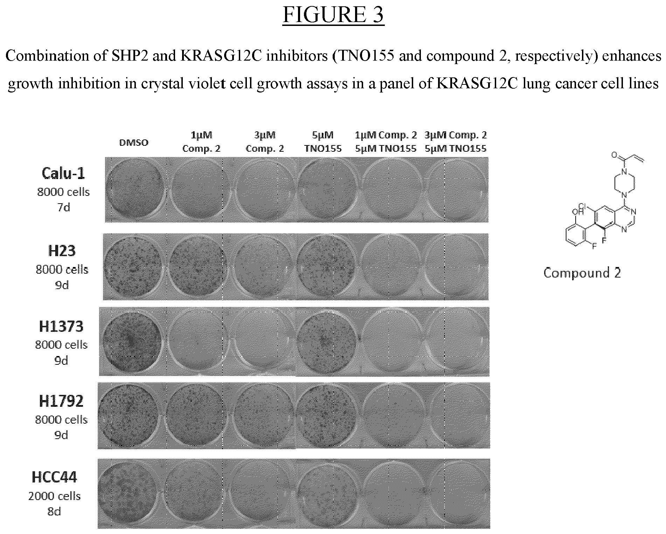

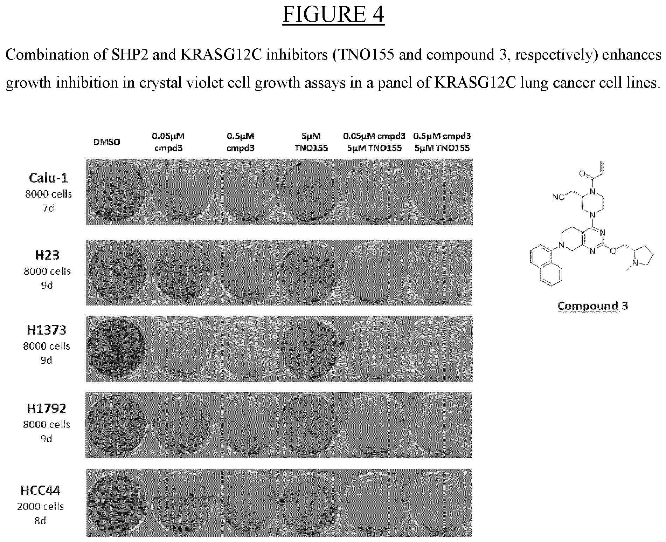

The results show that the combination of a SHP2 and KRASG12C inhibitor (SHP099 and cmpd1, respectively) enhances growth inhibition in crystal violet cell growth assays in a panel of KRASG12C lung cancer cell lines (figures 1 and 2). Further, the combination of SHP2 and KRASG12C inhibitors (TNO155 and compound 2 or 3, respectively) enhances growth inhibition in crystal violet cell growth assays in a panel of KRASG12C lung cancer cell lines (figure 3 and 4).

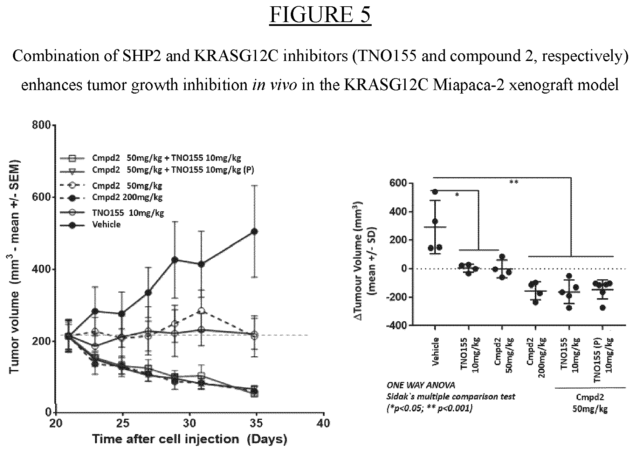

The results further show that the combination of SHP2 and KRASG12C inhibitors (TNO155 and cmpd2, respectively) enhances tumor growth inhibition *in vivo* in the KRASG12C Miapaca-2 xenograft model (figure 5).

It is understood that the Examples and embodiments described herein are for illustrative purposes only and that various modifications or changes in light thereof will be suggested to persons skilled in the art and are to be included within the spirit and purview of this application and scope of the appended claims.

## Claims

1. A compound which is (3S,4S)-8-(6-amino-5-((2-amino-3-chloropyridin-4-yl)thio)pyrazin-2-yl)-3-methyl-2-oxa-8-azaspiro[4.5]decan-4-amine, or a pharmaceutically acceptable salt thereof for use in the treatment of cancer, wherein the treatment comprises administration of a second therapeutic agent, and wherein the second therapeutic agent is a KRAS G12C inhibitor.

2. The compound or pharmaceutically acceptable salt thereof for use according to claim 1, wherein the cancer is selected from: esophageal or head and neck squamous cell carcinoma; colorectal, ovarian, pancreatic or non-small cell lung cancer; and renal cell carcinoma.

3. The compound or pharmaceutically acceptable salt thereof for use according to claim 1 or 2, wherein (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent are are administered simultaneously, separately or over a period of time.

4. The compound or pharmaceutically acceptable salt thereof for use according to any one of the preceding claims, wherein the KRAS G12C inhibitor is selected from 1-(4-(6-chloro-8-fluoro-7-(3-hydroxy-5-vinylphenyl)quinazolin-4-yl)piperazin-1-yl)prop-2-en-1-one--methane (1/2) (compound 1), (S)-1-(4-(6-chloro-8-fluoro-7-(2-fluoro-6-hydroxyphenyl)quinazolin-4-yl)piperazin-1-yl)prop-2-en-1-one (compound 2), and 2-((S)-1-acryloyl-4-(2-(((S)-1-methylpyrrolidin-2-yl)methoxy)-7-(naphthalen-1-yl)-5,6,7,8-tetrahydropyrido[3,4-d]pyrimidin-4-yl)piperazin-2-yl)acetonitrile (compound 3), or a pharmaceutically acceptable salt thereof.

5. The compound or pharmaceutically acceptable salt thereof for use according to any one of the preceding claims, wherein (3S,4S)-8-(6-amino-5-((2-amino-3-chloropyridin-4-yl)thio)pyrazin-2-yl)-3-methyl-2-oxa-8-azaspiro[4.5]decan-4-amine is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day.

6. The compound or pharmaceutically acceptable salt thereof for useaccording to claim 5 wherein the dose per day is on a 21 day cycle of 2 weeks on drug followed by 1 week off drug.

7. The compound or pharmaceutically acceptable salt thereof for use according toclaim 6 wherein the dose per day is 20 mg QD.
